# EUROPEAN PATENT APPLICATION

(11) **EP 4 197 452 A1**
(43) Date of publication of application: **21.06.2023**
(21) Application number: 22209364.3
(22) Date of filing: 24.11.2022
(51) Int. Cl.: A61B 17/00, A61F 2/04

(54) **IMPLANT FOR THE TREATMENT OF AN URINARY TRAKT**

(30) Priority: 15.12.2021 US 202163289829 P
(71) Applicant: Olympus Winter & Ibe GmbH, 22045 Hamburg (DE)
(72) Inventor: Jürgens, Thorsten, 21037 Hamburg (DE); Schweinberger, Henning, 21493 Basthorst (DE); Wosnitza, Thomas, 21337 Lüneburg (DE)
(74) Representative: Hoener, Matthias

(57) **Abstract**

The invention provides an implant that can be used to treat an urinary tract in a simple as well as reliable manner. This is achieved by allowing a wire structure (11) of an implant (10) to be inserted into a patient's urethra in a folded state with a distal end (19) first. Within the urethra, the wire structure (11) is deployed to treat the tissue. Denaturation occurs due to the ischaemic pressure of the wires (12) on the surrounding tissue. At least one further means for cutting off or incising tissue is positioned on the wire structure (11).

## Description

The invention relates to an implant according to the generic term of claim 1.

Various methods or techniques are known for the treatment of the urinary tract, especially *benign prostatic hyperplasia (BPH).* In a minimally invasive, particularly body-sparing treatment of BPH symptoms, a removable implant is temporarily placed in the urethra or in the prostatic part of the patient's urethra. Such an implant is a wire structure made of a shape memory alloy, such as nitinol. The wire structure is pushed into place by a catheter in a collapsed state, where it unfolds into its predetermined basic structure. This structure, which can be formed from three or four wires, is a basket structure. This basket structure expands the urethra. The expansion of the wire structure against the tissue of the urethra denatures the tissue of the urethra over the course of a few days. This denaturation of the tissue occurs due to the ischaemic pressure of the individual wires on the cells of the tissue, which leads to a reduced or complete lack of blood flow. As a result, the lack of blood flow leads to a lack of oxygen in the cells and finally to cell death. Within a few days, the tissue can be reduced to such an extent that the urine flow almost returns to normal. After completion of this treatment, the implant can be retrieved from the urethra by means of a catheter.

Through the technique described above, the tissue in the urethra can be treated in a very efficient way. Due to the enlargement of the prostate, the *lobus medius* (medial lobe) can also bulge far into the interior of the bladder. Due to this protrusion of the medial lobe, but also due to a possible enlargement of the medial lobe, it is conceivable that the bladder outlet becomes displaced. However, this displacement of the bladder outlet due to a protrusion and/or an enlargement of the medial lobe can also occur independently of an enlargement of the prostate. It is important to avoid obstruction of the bladder outlet for medical reasons. However, known methods and techniques for this are rather complex and traumatising for the patient. The technique described above is also not suitable for treating the urinary tract appropriately.

On this basis, the invention is based on the task of creating an implant with which the urinary tract can be treated in a simple and reliable manner.

A solution to this task is described by the features of claim 1. Accordingly, it is provided that an implant, in particular a removable implant, which consists of a wire structure with at least two wires, is insertable into the urethra of a patient in a folded state with a distal end first. Within the urethra, the wire structure is deployed to treat the tissue. The ischaemic pressure of the wires on the surrounding tissue causes the denaturation described above. The invention provides for at least one further means for cutting off or incising tissue to be positioned on the wire structure. This at least one further means is designed in such a way that it exerts a local ischaemic pressure on the medial lobe in the deployed state of the implant. Thus, in addition to the treatment of the urethral tissue by the further means, the medial lobe is also treated. At the end of the treatment, the tissue of the medial lobe is denatured due to the continuous pressure. The denaturation of the tissue causes space to be given for the further flow of urine. The fact that this further means is placed on a wire structure for the treatment of BPH symptoms allows the urinary tract to be treated in a particularly efficient, simple as well as gentle way.

Preferably, the invention provides that the means for cutting off or cutting in is a wire of a shape memory material or a thread. The use of a shape memory material, such as nitinol, is particularly advantageous because the shape of the means or the relative orientation of the means to the wire structure can be predetermined. Thus, after insertion through a catheter into the body, the implant unfolds back into the predetermined initial structure. This initial structure is optimally adapted to the given anatomical conditions, whereby a particularly efficient treatment of the tissue can be realised.

The use of a thread is technically particularly simple and inexpensive. For example, a thread can be tied around the tissue in any shape and removed again after the treatment is finished. In addition, a simple thread is particularly space-saving, which is especially relevant for minimally invasive treatments.

In particular, the present invention further provides that the wire is insertable into the urethra in a folded state relative to the implant and unfolds in the urethra to form a predetermined angle with the wire structure. This angle is such that the implant reaches the urethra as well as the medial lobe. Furthermore, the angle is such that the wire presses on the tissue of the medial lobe under a mechanical pretension. The lateral orientation of the wire relative to the wire structure can also be determined in this way. It has proven to be particularly advantageous if the additional wire lies on the same longitudinal axis as the wire structure. This relative orientation allows the implant to be positioned in a very precise manner and removed or pulled into the catheter after treatment.

Preferably, it is further conceivable that the means for cutting off or cutting in, in particular the wire or the thread, is interlaced with a wire of the wire structure. By this interweaving of the wire or the thread with the wire structure, a sufficiently strong connection can be made. Moreover, this way no further fastening means are necessary to connect the further means to the wire structure. Through this interweaving, the further means is motion-coupled with the wire structure, whereby the prescribed combined treatment of the urethra as well as the medial lobe can be realised.

It is further conceivable according to the invention that the wire is bent at least in sections, wherein the wire is convexly bent relative to a longitudinal axis of the implant so that the bent wire can wrap around the medial lobe. The described shape of the wire allows the wire to exert pressure on the medial lobe along its entire length. This allows the treatment or denaturation of the tissue to be done in a very efficient way. Cutting the tissue can also be carried out in a particularly efficient way due to this shape, as a pre-tension is also exerted on the tissue due to the convex shape.

A further advantageous embodiment of the invention may provide that the thread is formed as a loop, which loop may be placed around the central flap. By applying pressure of the thread to the tissue of the medial lobe, the above-described denaturation of the tissue takes place, thus reducing the size of the medial lobe. Since the thread is very flexible, it can adapt to the shape of the medial lobe in a very effective way. By changing the size of the loop, the necessary constriction of the vessels can be achieved. By increasing the diameter, the central flap can be released again and the loop or thread removed.

Furthermore, it is conceivable according to the invention that one end of the loop is firmly connected to the implant and the other end is loosely guided to a proximal end of the implant, wherein the size of the diameter of the loop can be changed by pulling on the loose end and wherein in particular the loose end can be detachably fixed to the wire structure during treatment. The fixed connection of the one end can be made, for example, by reducing, gluing or crimping. The loose end can be moved either manually or via an auxiliary instrument. Pulling on the loose end tightens the loop so that pressure is applied to the medial lobe. By releasing the loose end, the implant and suture can be removed from the urinary tract without leaving any residue.

For guiding the thread on the wire structure, it is conceivable that at least one or more sleeves are arranged on the wire structure through which the thread, in particular both ends of the loop, can be guided. In this case, the sleeves or a longer hose-like or tube-like sleeve are guided over a wire of the wire structure. It is conceivable that these sleeves are fixed at certain positions of the wire so that they cannot slip along the wire structure. Alternatively, it is also conceivable that such sleeves are assigned to several wires, so that sleeves of one wire can receive one end of the thread and sleeves of another wire can receive the other end of the thread. These sleeves allow the thread to be directed and moved in a very defined way. Furthermore, it can be ensured that the thread is guided along the wire structure in a defined manner and does not become knotted with it. As the wires of the wire structure are pressed against the tissue of the urethra during the treatment, the sleeves can also ensure that the thread can be moved during the treatment to change the size of the loop.

A further embodiment of the invention may provide that a further wire is arranged on the wire structure and that this wire is oriented in such a way that it lifts the medial lobe during treatment. This lifting of the medial lobe can already open the bladder outlet for the undisturbed flow of urine. In addition, this lifting makes it easier to reach the medial lobe with the wire or sling. This wire can also be made of a shape memory material, especially nitinol. The wire unfolds in the urethra in the same way as the wire structure, except that it points in a distal direction and has a protruding distal end with which the medial lobe can be pushed up. This other wire may be attached to the wire structure in the same way as the thread or wire for cutting or incising tissue.

A preferred embodiment of the present invention is explained in more detail below with reference to the drawing. In this show:
- Fig. 1: an illustration of a first embodiment of an implant,
- Fig. 2: an illustration of a further embodiment of an implant, and
- Fig. 3: another illustration of the embodiment of the implant according to Fig. 2.

The figures schematically illustrate possible embodiments of the invention. It should be expressly noted that the invention is not intended to be limited to these embodiments. Rather, it is intended that the invention can also be realised by other embodiments.

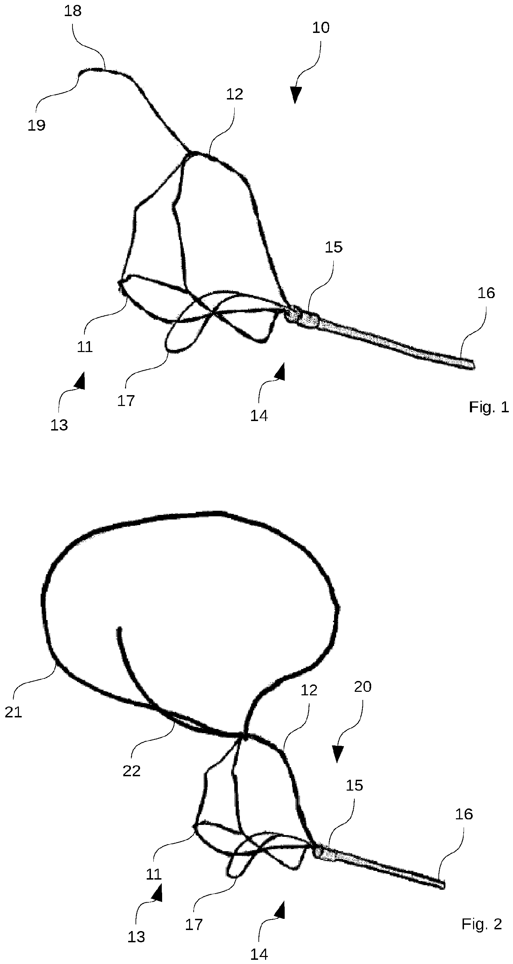

Fig. 1 shows an implant 10 consisting essentially of a wire structure 11. The wire structure shown in Fig. 1 forms a basket which is stretched by three wires 12. The wires 12 are twisted together in sections. At a distal end 13, the wires 12 are arcuate and each runs back to a proximal end 14, where they are brought together in a connector 15. This connector 15 positioned at the proximal end 14 also serves to contact a handling means 16. This handling means 16 can be formed as a thread or rod or be part of a catheter.

Furthermore, the implant 10 has an anchoring clamp 17. This anchoring clamp 17 is arch-shaped and serves to anchor or fix the implant 10 during treatment in the urethra, which is not shown. As explained above, the implant 10 is inserted into the patient's urethra for treatment in a folded state through a catheter not shown. At its destination, the implant 10 unfolds into the shape shown in Fig. 1. As it does so, the wires 12 press on the surrounding tissue of the urethra, causing the tissue to denature. After the treatment, the implant 10 can be pulled out of the urethra through the catheter with the aid of the handling means 16. In doing so, the implant 10 folds back into the catheter. In order for the implant 10 to unfold at the appropriate location in the urethra, it is made of a shape memory material, namely nitinol. However, in addition to this aforementioned material, other shape memory materials are also conceivable.

The embodiment example shown in Fig. 1 now has a further wire 18. This wire 18 is stretched in the distal direction in the unfolded state of the implant 10. In this case, the wire 18 is attached to one of the wires 12 of the wire structure 11. During placement or during the folded state, this wire 18 is swivelled in within the folded wire structure 11. When the implant 10 is unfolded, the wire 18 pivots distally forward to form an angle with the other wires 12. The wire 18 is slightly bent, forming a concave curvature relative to a longitudinal axis of the implant 10. This curvature allows the wire 18 to wrap around the central lobe in a very efficient manner.

The distal end 19 of the wire may be pointed or sharp. During the treatment of the urethra by the wire structure 11, the wire 18 continuously presses on the not shown medial lobe or tissue. This pressurisation causes the tissue to denature. When the implant 10 is withdrawn, the distal end 19 of the wire 18 travels along or through the medial lobe, which can cause an incision. Both the denaturation and the incision of the medial lobe result in the pathway being reopened for urinary flow.

An alternative embodiment of the implant 20 shown in Fig. 2 provides that a thread 21 is attached to at least one of the wires 12. One end of this thread 21 is firmly attached to the wire structure 11. Another end of the thread 21 is also associated with the wire structure 11, but is free to move. Here, this free or loose end of the thread 21 is guided along a wire 12 to the proximal end 14 of the implant 20. There, the free end of the thread 21 can be grasped manually or by a tool.

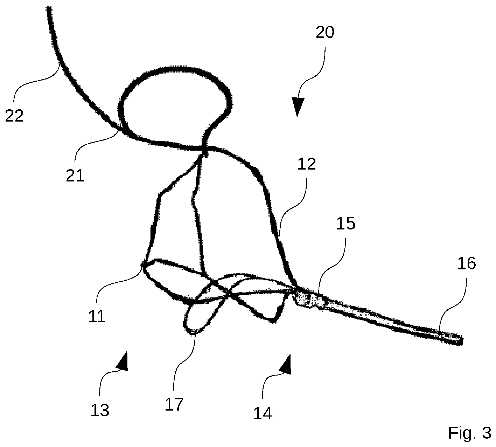

According to the invention, it is envisaged that the loop formed by the thread 21 is passed around the centre flap and that a pull on the loose end of the thread 21 causes the centre flap tissue to be strangulated (Fig. 3). This strangulation can also cause denaturation of the tissue. If necessary, the tensile force of the thread 21 is increased regularly to accelerate the regression of the medial lobe. At the end of the treatment, the tensile force on the thread 21 is removed. When the implant 20 is pulled out of the patient, the central flap is also released from the thread 21.

The thread 21 can either be interwoven with the wires 12 of the wire structure 11 or guided in sleeves not shown. It is conceivable that a wire 12 is guided in a sleeve in which the thread 21 can then also be guided. Both the fixed and the loose end of the thread 21 can be guided in this sleeve. Alternatively, it is also conceivable that the fixed end of the thread 21 is arranged outside the sleeve and only the loose end of the thread 21 is guided through the sleeve. A further embodiment example may provide that a wire 12 of the wire structure 11 is associated with a plurality of sleeves fixed to the wires 12, through which the thread 21 is guided with at least one end.

The embodiment example of the implant 20 according to Figs. 2 and 3 also has a further wire 22. This wire 22 is also attached to the wire structure 11 and is bent obliquely upwards in the distal direction. The wire 22 may preferably be made of a shape memory material and, similarly to the thread 21, is attached at one end to at least one of the wires 12. In the folded state of the implant 20, this wire 22 is also located folded within the wire structure 11. When the wire structure 11 is unfolded, the wire 22 moves out of the wire structure 11 and serves to push up the centre flap during treatment so that it does not close the bladder opening. Furthermore, this pushing up of the medial lobe also serves to place the thread 21 or loop around the medial lobe. Due to the bent shape of the wire 22, it has a certain mechanical pre-tension, through which a resilient force can be exerted on the medial lobe. After the treatment is finished, the wire 22 together with the implant 20 is pulled out of the urethra.

### List of Reference Numerals:

- 10: Implant
- 11: Wire structure
- 12: Wire
- 13: Distal end
- 14: Proximal end
- 15: Connector
- 16: Handling means
- 17: Anchoring clamp
- 18: Wire
- 19: Distal end
- 20: Implant
- 21: Thread
- 22: Wire

## Claims

1. Implant (10, 20), in particular a removable implant (10, 20), for the treatment of an urinary tract of a person by applying a local ischemic pressure to the tissue of urinary organs, in particular the urethra and/or the medial lobe, by means of a wire structure (11) with at least two wires (12), wherein the implant (10, 20) being insertable into the urethra in a folded state with a distal end (19) first and unfolding in the urethra to the wire structure (11) for treatment of the tissue, **characterized by** at least one further means for constricting or incising tissue, said means being positioned on the wire structure (11) such that it exerts a local ischemic pressure on a medial lobe of the person.

2. Implant (10, 20) according to claim 1, **characterised in that** the means for cutting off or cutting in is a wire (18) of a shape memory material or a thread (21).

3. Implant (10, 20) according to claim 2, **characterized in that** the wire (18) is insertable into the urethra in a folded state relative to the implant (10, 20) and unfolds in the urethra to include a predetermined angle with the wire structure (11).

4. Implant (10, 20) according to any one of the preceding claims, **characterised in that** the means for cutting off or cutting in, in particular the wire (18) or the thread (21), is interlaced with a wire (12) of the wire structure (11).

5. Implant (10, 20) according to any one of claims 2 to 4, **characterised in that** the wire (18) is bent at least in sections, the wire (18) being convexly bent relative to a longitudinal axis of the implant (10, 20) so that the bent wire (18) can wrap around the medial lobe.

6. Implant (10, 20) according to claim 2, **characterised in that** the thread (21) is formed as a loop, which loop can be placed around the medial lobe.

7. Implant (10, 20) according to claim 6, **characterised in that** a diameter of the loop is variable.

8. Implant (10, 20) according to claim 6 or 7, **characterised in that** one end of the loop is firmly connected to the implant (10, 20) and the other end is loosely guided to a proximal end (14) of the implant (10, 20), wherein the size of the diameter of the loop can be changed by pulling on the loose end of the thread (21) and wherein in particular the loose end of the thread (21) can be releasably fixed to the wire structure (11) during treatment.

9. Implant (10, 20) according to any one of claims 6 to 8, **characterised in that** the thread (21) is guided in at least one sleeve on the wire structure (11).

10. Implant (10, 20) according to any one of the preceding claims, **characterised in that** a further wire (22) is arranged on the wire structure (11) and this wire (22) is oriented in such a way that it raises the medial lobe during treatment.

11. Implant (10, 20) according to one of the previous claims, **characterised in that** all wires (12) and threads (21) are brought together at a proximal end (14) of the implant (10, 20), preferably at a crimping point, and the loose wires (12) and threads (21) can be fixed there or guided outwards through the urethra.
